(19) Europäisches Patentamt
European Patent Office
Office européen des brevets

(11) EP 1 165 026 B1

(12) **FASCICULE DE BREVET EUROPEEN**

(45) Date de publication et mention de la délivrance du brevet:
**12.05.2004 Bulletin 2004/20**

(21) Numéro de dépôt: **00907709.0**

(22) Date de dépôt: **24.02.2000**

(51) Int Cl.[7]: **A61K 7/13**, A61K 7/06

(86) Numéro de dépôt international:
**PCT/FR2000/000456**

(87) Numéro de publication internationale:
**WO 2000/057848 (05.10.2000 Gazette 2000/40)**

(54) **PROCEDE DE TEINTURE D'OXYDATION UTILISANT LA N-ACETYLCYSTEINE A TITRE D'AGENT REDUCTEUR ET UNE LACCASE A TITRE D'AGENT OXYDANT**

VERFAHREN ZUM OXIDATIVEN FÄRBEN MIT N-ACETYLCYSTEIN ALS REDUKTIONSMITTEL UND LACASSE ALS OXIDATIONSMITTEL

OXIDATION DYEING METHOD USING N-ACETYLCYSTEINE AS A REDUCING AGENT AND LACCASE AS AN OXIDATING AGENT

(84) Etats contractants désignés:
**AT BE CH CY DE DK ES FI FR GB GR IE IT LI LU MC NL PT SE**

(30) Priorité: **26.03.1999 FR 9903829**

(43) Date de publication de la demande:
**02.01.2002 Bulletin 2002/01**

(73) Titulaire: **L'OREAL**
**75008 Paris (FR)**

(72) Inventeur: **PLOS, Grégory**
**F-75015 Paris (FR)**

(74) Mandataire: **Casalonga, Axel**
**BUREAU D.A. CASALONGA - JOSSE**
**Paul-Heyse-Strasse 33**
**80336 München (DE)**

(56) Documents cités:
**EP-A- 0 429 855**
**EP-A- 0 673 641**
**EP-A- 0 716 846**
**EP-A- 1 142 562**
**EP-A- 1 142 563**
**WO-A-97/19998**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 165 026 B1

Printed by Jouve, 75001 PARIS (FR)

## Description

La présente invention concerne un procédé de teinture d'oxydation des fibres kératiniques, et en particulier des fibres kératiniques humaines telles que les cheveux, mettant en oeuvre des compositions comprenant, dans un milieu approprié pour la teinture, au moins un précurseur de colorant d'oxydation, éventuellement, un ou plusieurs coupleurs, et de la N-acétylcystéine comme agent réducteur, et au moins une laccase à titre d'agent oxydant.

Il est connu de teindre les fibres kératiniques, et en particulier les cheveux, avec des compositions de teinture contenant des précurseurs de colorants d'oxydation, généralement appelés "bases d'oxydation", en particulier des ortho- ou para-phénylènediamines, des ortho- ou para-aminophénols et des bases hétérocycliques.

Les précurseurs de colorants d'oxydation sont des composés initialement peu ou pas colorés qui développent leur pouvoir tinctorial au sein du cheveu en présence d'un agent oxydant. L'agent oxydant utilisé est généralement le peroxyde d'hydrogène. La formation des composés colorés résulte, soit d'une condensation des "bases d'oxydation" sur elles-mêmes, soit d'une condensation des "bases d'oxydation" sur des composés modificateurs de coloration, ou "coupleurs", qui sont généralement présents dans les compositions tinctoriales utilisées en teinture d'oxydation et qui sont représentés plus particulièrement par des méta-phénylènediamines, des méta-aminophénols et des méta-diphénols et certains composés hétérocycliques.

La variété des molécules mises en jeu, qui sont constituées d'une part par "les bases d'oxydation" et, d'autre part, par les "coupleurs", permet l'obtention d'une palette riche en coloris.

La coloration d'oxydation des fibres kératiniques peut également être réalisée à l'aide de systèmes oxydants différents du peroxyde d'hydrogène tel que des systèmes enzymatiques. Ainsi, il a déjà été proposé dans le brevet US 3,251,742, la demande de brevet FR-A-2 112 549, FR-A-2 694 018, EP-A-0 504 005, WO 95/07998, WO 95/33836, WO 95/33837, WO 96/00290, WO 97/19998 et WO 97/19999 de teindre les fibres kératiniques avec des compositions comprenant au moins un colorant d'oxydation en association avec des enzymes du type laccases, lesdites compositions étant mises en contact avec l'oxygène de l'air. En effet, il a été observé que l'eau oxygénée pouvait provoquer une dégradation de la fibre capillaire, et, en outre, une attaque partielle de la mélanine du cheveu, ce qui conduit à l'éclaircissement de la fibre.

La demande EP-A-0 716 846 divulgue des compositions de teinture d'oxydation contenant une uricase en tant qu'enzyme d'oxydation.

Les demandes EP-A-1 142 561 et EP-A-142 562, ayant une date de priorité antérieure à celle la présente demande mais publiées seulement après celle-ci, divulguent des compositions de teinture d'oxydation contenant, en tant qu'enzyme d'oxydation, une laccase stabilisée respectivement par la présence d'eau contenant au moins 0,1 % de dioxyde de carbone ou par un conditionnement à l'abri de l'air.

Pour pouvoir conserver les précurseurs de colorants d'oxydation et les coupleurs, il est nécessaire de les associer à un réducteur.

Cependant, la demanderesse a constaté que ces réducteurs freinent généralement la montée des colorants sur les fibres, ce qui se traduit par des nuances moins lumineuses et des colorations moins puissantes.

Pour obtenir une chromaticité équivalente, il est alors nécessaire d'utiliser des quantités plus importantes de colorants.

De plus, de nombreux réducteurs utilisés jusqu'à présent, possèdent une action inhibitrice de l'activité de la laccase.

Après d'importantes recherches effectuées dans ce domaine, la demanderesse vient de découvrir que l'utilisation de N-acétylcystéine comme agent réducteur quand une laccase est utilisée comme agent oxydant permettait de résoudre les problèmes ci-dessus mentionnés.

En effet, il a été constaté que la N-acétylcystéine n'inhibait pas l'activité de la laccase; de plus, il a été constaté de façon surprenante que le mélange ainsi produit ne freinait pas la montée des colorants d'oxydation sur les cheveux.

Ces compositions donnent par ailleurs naissance à des nuances plus chromatiques (plus lumineuses) et à des colorations plus puissantes par rapport à des compositions équivalentes contenant des réducteurs et des agents oxydants habituels.

Les colorations obtenues présentent par ailleurs une bonne résistance à la transpiration, à la lumière et aux shampooings.

L'invention permet également de diminuer la quantité de matières actives colorantes utilisées dans les compositions de teinture par rapport aux techniques classiques et connues de l'art antérieur.

La présente invention a ainsi pour objet l'utilisation de N-acétylcystéine à titre d'agent réducteur et d'une laccase à titre d'agent oxydant pour la teinture oxydative.

Un autre objet de l'invention concerne un procédé de teinture des fibres kératiruques, et en particulier des fibres kératiniques humaines telles que les cheveux, consistant:

- à appliquer sur les fibres une composition de teinture (A) contenant, dans un milieu approprié pour la teinture, au

moins un précurseur de colorant d'oxydation et, éventuellement, un ou plusieurs coupleurs et à titre d'agent réducteur, de 0,01 à 0,25 % en poids, par rapport au poids total de la composition (A), de N-acétylcysteine, et

- à révéler en présence d'air la couleur en milieu alcalin, neutre ou acide à l'aide d'au moins une laccase à titre d'agent oxydant, la laccase étant incorporée dans la composition (A), dans ce cas stockée à l'abri de l'air, ou dans une composition (B), les compositions (A) et (B) étant dans ce second cas mélangées immédiatement avant l'emploi ou appliquées l'une après l'autre sur les fibres kératiniques.

La ou les laccases utilisées dans le procédé conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.

Parmi les laccases d'origine végétale utilisables selon l'invention, on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera Indica, Schinus molle ou Pleiogynium timoriense, dans les extraits des Podocarpacées, de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

Parmi les laccases d'origine fongique éventuellement obtenues par biotechnologie utilisables selon l'invention, on peut citer la ou les laccases issues de Polyporus versicolor, de Rhizoctonia praticola et de Rhus vemicifera comme indiquées dans les demandes FR-A-2 112 549 et EP-A-504005; celles décrites dans les demandes de brevet WO95/07988, WO95/33836, WO95/33837, WO 96/00290, WO97/19998 et WO 97/19999, comme par exemple celles issues de Scytalidium, de Polyporus pinsitus, de Myceliophtora thermophila, de Rhizoctonia solani, de Pyricularia orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.

On choisira plus préférentiellement les laccases d'origine fongique éventuellement obtenues par biotechnologie.

L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de 1mmole de syringaldazine par minute à pH 5,5 à 30°C. L'unité u correspond à la quantité d'enzyme produisant un delta d'absorbance à 530 nm de 0,001 par minute en utilisant la syringaldazine comme substrat, à 30°C et à pH 6,5.

L'activité enzymatique des laccases de l'invention peut aussi être définie à partir de l'oxydation de la paraphénylènediamine. L'unité ulac correspond à la quantité d'enzyme produisant un delta d'absorbance à 496,5nm de 0,001 par minute en utilisant la paraphénylènediamine comme substrat (64 mM) à 30°C et à pH 5.

Selon l'invention, on préfère déterminer l'activité enzymatique en unités ulac.

Les quantités de laccase utilisées dans les compositions de l'invention varieront en fonction de la nature de la laccase choisie. De façon préférentielle, elles varieront de 0,5 à 3000 lacu, ou de 1000 à $6.10^7$ unités u; ou de 20 à $3.10^6$ unités ulac pour 100g de composition appliquée sur les cheveux.

Les précurseurs de colorants d'oxydation utilisables dans le cadre de la présente invention sont choisis parmi ceux classiquement connus en teinture d'oxydation. On peut citer notamment les ortho-phénylènediamines, les paraphénylènediamines telles celles de formule (I) suivante et les sels d'addition d'un acide de ces composés

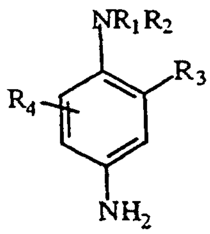

(I)

dans laquelle

$R_1$ représente un atome d'hydrogène, un radical alkyle en $C_{1-4}$, monohydroxyalkyle en $C_{1-4}$, polyhydroxyalkyle en $C_{2-4}$ ou 4'-aminophényle,
$R_2$ représente un atome d'hydrogène, un radical alkyle en $C_{1-4}$, monohydroxyalkyle en $C_{1-4}$ ou polyhydroxyalkyle en $C_{2-4}$,
$R_3$ représente un atome d'hydrogène, un atome d'halogène tel qu'un atome de chlore, un radical alkyle en $C_{1-4}$, sulfo, carboxy, monohydroxyalkyle en $C_{1-4}$ ou hydroxyalcoxy en $C_{1-4}$,
$R_4$ représente un atome d'hydrogène ou un radical alkyle en $C_{1-4}$.

Parmi les para-phénylènediamines de formule (I) ci-dessus, on peut citer en particulier la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N,N-diméthyl-para-phénylènediamine, la N,N-diéthyl-para-phénylènediamine, la N,N-dipropyl-para-phénylènediamine, la 4-amino-N,N-diéthyl-3-méthylaniline, la N,N-bis(β-hydroxyéthyl)-para-phénylènediamine, la 4-amino-N,N-bis(β-hydroxyéthyl)-3-méthyl-aniline, la 4-amino-3-chloro-N,N-bis(β-hydroxyéthyl)-aniline, la 2-β-hydroxyéthyl-para-phénylènediamine, la 2-fluoro-para-phénylènediamine, la 2-isopropyl-para-phénylène, la N-(β-hydroxypropyl)-para-phénylènediamine, la 2-hydroxyméthyl-para-phénylènediamine, la N,N-diméthyl-3-méthyl-para-phénylènediamine, la N-éthyl-N-β-hydroxyéthyl)-para-phénylènediamine, la N-(β,γ-dihydroxypropyl)-paraphénylènediamine, la N-(4'-aminophényl)-para-phénylènediamine, la N-phényl-para-phénylènediamine, la 2-β-hydroxyéthyloxy-para-phénylènediamine et les sels d'addition d'acide de ces composés.

Parmi les para-phénylènediamines de formule (I) ci-dessus, on préfère tout particulièrement la para-phénylènediamine, la para-toluylènediamine, la 2-isopropyl-para-phénylènediamine, la 2-β-hydroxy-éthyl-para-phénylèndiamine, la 2-β-hydroxyéthyloxy-para-phénylènediamine, la 2,6-diméthyl-para-phénylènediamine, la 2,6-diéthyl-para-phénylènediamine, la 2,3-diméthyl-para-phénylènediamine, la N,N-bis-(β-hydroxyéthyl)-para-phénylènediamine, la 2-chloro-para-phénylènediamine et les sels d'addition d'acide de ces composés.

- les bis-phénylalkylènediamines telles celles de formule (II) :

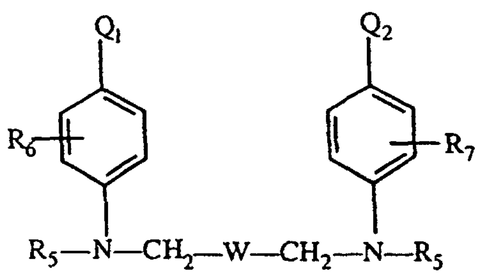

(II)

dans laquelle

$Q_1$ et $Q_2$, identiques ou différents, représentent un radical hydroxyle ou $NHR_8$ dans lequel $R_8$ représente un atome d'hydrogène ou un radical alkyle en $C_{1-4}$,
$R_5$ représente un atome d'hydrogène, un radical alkyle en $C_{1-4}$, monohydroxyalkyle en $C_{1-4}$, polyhydroxyalkyle en $C_{2-4}$ ou aminoalkyle en $C_{1-4}$ dont le groupe amino peut être substitué,
$R_6$ et $R_7$, identiques ou différents, représentent un atome d'hydrogène ou d'halogène ou un radical alkyle en $C_{1-4}$,
W représente un radical choisi dans le groupe formé par les radicaux suivants :

$-(CH_2)_n-$ ; $-(CH_2)_m-O-(CH_2)_m$ ; $-(CH_2)_m-CHOH-(CH_2)_m-$ et
$-(CH_2)_m-N(CH_3)-(CH_2)_m-$ ;

dans lesquels n est un nombre entier compris entre 0 et 8 inclusivement et m est un nombre entier compris entre 0 et 4 inclusivement, et les sels d'addition d'acide de tels composés.

Parmi les bis-phénylalkylènediamines de formule (II) ci-dessus, on peut citer en particulier le N,N'-bis(β-hydroxyéthyl)-N,N'-bis(4-aminophényl)-1,3-diamino-2-propanol, la N,N'-bis(β-hydroxyéthyl)-N,N'-bis(4-aminophényl)-éthylènediamine, la N,N'-bis(4-aminophényl)-tétraméthylènediamine, la N,N'-bis(β-hydroxyéthyl)-N,N'-bis(4-aminophényl)-tétraméthylènediamine, la N,N'-bis(4-méthylaminophényl)-tétraméthylènediamine, la N,N'-bis(β-hydroxyéthyl) -N,N'-bis(4-aminophényl)-tétraméthylènediamine, la N,N'-bis(éthyl)-N,N'-bis(4-amino-3-méthylphényl)-éthylènediamine, et les sels d'addition d'acide de ces composés.

On recommande en particulier parmi ces bis-phénylalkylènediamines de formule (II) le N,N'-bis(β-hydroxyéthyl)-N,N'-bis(4'-aminophényl)-1,3-diamino-2-propanol ou l'un de ses sels d'addition d'un acide.

- les para-aminophénols tels ceux répondant à la formule (III) :

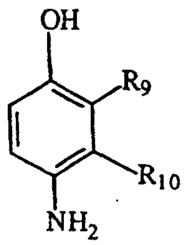

(III)

dans laquelle

$R_9$ représente un atome d'hydrogène, un radical alkyle en $C_{1-4}$, monohydroxyalkyle en $C_{1-4}$, (alcoxy en $C_{1-4}$)-(alkyle en $C_{1-4}$) ou aminoalkyle en $C_{1-4}$, ou hydroxy(alkyle en $C_{1-4}$)-aminoalkyle en $C_{1-4}$);
$R_{10}$, représente un atome d'hydrogène ou de fluor, un radical alkyle en $C_{1-4}$, monohydroxyalkyle en $C_{1-4}$, polyhydroxyalkyle en $C_{2-4}$, aminoalkyle en $C_{1-4}$, cyano(alkyle en $C_{1-4}$) ou (alcoxy en $C_{1-4}$)-(alkyle en $C_{1-4}$), et les sels d'addition d'acide de tels composés, avec la réserve qu'au moins un des radicaux $R_9$ ou $R_{10}$ représente un atome d'hydrogène.

Parmi les para-aminophénols de formule (III) ci-dessus, on peut citer notamment le para-aminophénol, le 4-amino-3-méthylphénol, le 4-amino-3-fluorophénol, le 4-amino-3-hydroxyméthyl-phénol, le 4-amino-2-méthyl-phénol, le 4-amino-2-hydroxyméthylphénol, le 4-amino-2-méthoxyméthyl-phénol, le 4-amino-2-aminométhylphénol, le 4-amino-2-(β-hydroxyéthyl-aminométhyl)-phénol, et les sels d'addition d'acide de ces composés.

- les ortho-aminophénols utilisables à titre de bases d'oxydation dans le cadre de la présente invention sont notamment choisis parmi le 2-aminophénol, le 2-amino-1-hydroxy-5-méthylbenzène, le 2-amino-1-hydroxy-6-méthylbenzène, le 5-acétamido-2-aminophénol et les sels d'addition d'acide de ces composés;
- les bases hétérocycliques utilisables à titre de bases d'oxydation dans le cadre de la présente invention sont notamment choisies parmi les dérivés pyridiniques, les dérivés pyrimidiniques, les dérivés pyrazoliques et les sels d'addition d'acide de ces composés.

Parmi les dérivés pyridiniques, on peut plus particulièrement citer les composés décrits par exemple dans les brevets GB-1 026 978 et GB-1 153 196, comme la 2,5-diaminopyridine et les sels d'addition d'acide de tels composés.

Parmi les dérivés pyrimidiniques, on peut citer en particulier les composés décrits par exemple dans le brevet allemand DE-2 359 399 ou les brevets japonais JP-88-169 571, comme la 2,4,5,6-tétraaminopyrimidine, la 4-hydroxy-2,5,6,-triaminopyrimidine et les sels d'addition d'acide de tels composés.

Parmi les dérivés pyrazoliques, on peut plus particulièrement citer les composés décrits dans les brevets DE-3 843 892, DE-4 133 957 et demandes de brevet WO-94/08969 et WO-94/08970 comme le 4,5-diamino-1-méthylpyrazole, le 3,4-diamino-pyrazole, le 4,5-diamino-1-(4'-chlorobenzyl)-pyrazole et les sels d'addition d'acide de ces composés.

Selon l'invention, le ou les précurseurs de colorants d'oxydation représentent de préférence de 0,0005 à 12 % en poids du poids total de la composition (A) et encore mieux de 0,005 à 6 % en poids environ.

Les coupleurs utilisables dans le procédé de teinture selon l'invention sont ceux classiquement utilisés dans les compositions de teinture d'oxydation, c'est-à-dire des méta-phénylènediamines, des méta-aminophénols et des méta-diphénols (résorcinols), les dérivés mono- ou polyhydroxylés du naphtalène, le sésamol et ses dérivés et des composés hétérocycliques tels que, par exemple, les coupleurs indoliques, les coupleurs indoliniques, les coupleurs pyridiniques et les sels d'addition d'acide de tels composés.

Ces coupleurs peuvent notamment être choisis parmi le 2-méthyl-5-amino-phénol, le 5-N-(β-hydroxyéthyl)-amino-2-méthylphénol, le 3-aminophénol, le 1,3-dihydroxybenzène, le 1,3-dihydroxy-2-méthylbenzène, le 4-chloro-1,3-dihydroxybenzène, le 1-(β-hydroxyéthoxy)-2,4-diaminobenzène, le 2-amino-4-(β-hydroxyéthylamino)-1-méthoxybenzène, le 1,3-diaminobenzène, le 1,3-bis(2,4-diaminophénoxy)-propane, le sésamol, l'α-naphtol, le 6-hydroxyindole,

le 4-hydroxyindole, le 4-hydroxy-N-méthylindole, la 6-hydroxyindoline, la 2,6-dihydroxy-4-méthylpyridine, la 1-H-3-méthylpyrazol-5-one, la 1-phényl-3-méthyl-pyrazol-5-one et les sels d'addition d'acide de tels composés.

Lorsqu'ils sont présents, ces coupleurs représentent de préférence d'environ 0,0001 à 10 % en poids du poids total de la composition (A), et en particulier d'environ 0,005 à 5 % en poids.

D'une manière générale, les sels d'addition d'un acide des composés chromogènes, à savoir les bases d'oxydation et les coupleurs, sont choisis notamment parmi les chlorhydrates, les bromhydrates, les sulfates, les tartrates, les lactates et les acétates.

La composition (A) peut contenir, en plus des précurseurs de colorants d'oxydation définis ci-dessus et des éventuels coupleurs associés, des colorants directs pour enrichir les nuances en reflets. Ces colorants directs peuvent notamment être choisis parmi les colorants nitrés, azoïques ou anthraquinoniques.

La composition (A) et/ou la composition (B) peuvent en outre contenir au moins un polymère substantif cationique ou amphotère tel que ceux définis dans EP-A-0 673 641, parmi lesquels on préfère avantageusement mettre en oeuvre :

- les polymères poly(ammonium quaternaire) préparés et décrits dans le brevet français 2 270 846, constitués de motifs récurrents répondant à la formule (IV) suivante :

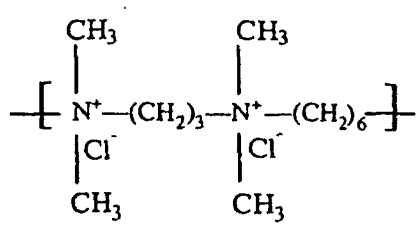

(IV)

et dont la masse molaire moyenne en poids, déterminée par chromatographie par perméation de gel, est comprise entre 9500 et 9900 ;

- les polymères poly(ammonium quaternaire) préparés et décrits dans le brevet français 2 270 846, constitués de motifs récurrents répondant à la formule (V) suivante :

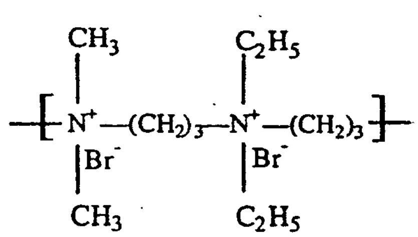

(V)

et dont la masse molaire moyenne en poids, déterminée par chromatographie par perméation de gel, est d'environ 1200.

Le milieu de la composition (A) approprié pour la teinture est de préférence un milieu aqueux constitué majoritairement d'eau et contenant, éventuellement, des solvants organiques acceptables sur le plan cosmétique, parmi lesquels figurent des alcools tels que l'alcool éthylique, l'alcool isopropylique, l'alcool benzylique et l'alcool phényléthylique; des glycols ou éthers de glycols tels que les éthers monométhylique, monoéthylique et monobutylique d'éthylèneglycol, le propylèneglycol ou ses éthers tels que l'éther monométhylique de propylèneglycol; le butylèneglycol; le dipropylèneglycol ainsi que les alkyléthers de diéthylèneglycol comme par exemple l'éther monométhylique ou monobutylique de diéthylèneglycol, en des concentrations comprises entre environ 0,5 et 20 % en poids, de préférence entre environ 2 et 10 % en poids, par rapport au poids total de la composition.

La composition (A) peut encore contenir une quantité efficace d'autres agents utilisés couramment dans le domaine de la teinture d'oxydation. Ces adjuvants sont par exemple des agents séquestrants, des agents de conditionnement du cheveu et en particulier des silicones, des agents conservateurs, des agents opacifiants etc., et éventuellement des agents tensio-actifs anioniques, non-ioniques, amphotères ou des mélanges de ceux-ci.

Bien entendu, l'homme de métier veillera à choisir le ou les éventuels composés complémentaires mentionnés ci-avant, d'une manière telle que les propriétés avantageuses attachées intrinsèquement à la composition tinctoriale selon l'invention ne soient pas, ou pratiquement pas, altérées par la ou les adjonctions envisagées.

Les valeurs du pH des compositions (A) et (B) peuvent notamment être choisies de manière à ce que la valeur du pH de la composition prête à l'emploi, résultant du mélange de la composition tinctoriale (A) et de la composition oxydante (B), soit généralement comprise entre 3 et 11, de préférence entre 4 et 9 et encore plus préférentiellement entre 6 et 8. Elles peuvent être ajustées au moyen d'agents acidifiants ou alcalinisants bien connus dans la technique de teinture d'oxydation des fibres kératiniques.

On peut citer parmi les agents alcalinisants, par exemple l'ammoniaque, les carbonates alcalins, les alcanolamines telles que les mono-, di- et triéthanolamines ainsi que leurs dérivés, les hydroxydes de sodium ou de potassium et les composés de formule (VI) suivante :

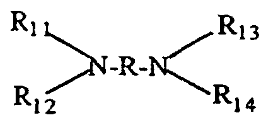

(VI)

dans laquelle R est un résidu propylène éventuellement substitué par un groupement hydroxyle ou un radical alkyle en $C_{1-4}$; $R_{11}$, $R_{12}$, $R_{13}$ et $R_{14}$, identiques ou différents, représentent un atome d'hydrogène ou un radical alkyle en $C_{1-4}$ ou hydroxyalkyle en $C_{1-4}$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique ou des acides sulfoniques.

Un autre objet de la présente invention est une composition prête à l'emploi pour la teinture des fibres kératiniques contenant la laccase et le
ou les précurseurs de colorants d'oxydation ou susceptible d'être obtenue par mélange des compositions (A) et (B) ci-dessus définies.

L'invention a également pour objet un procédé de teinture des fibres kératiniques et en particulier des fibres kératiniques humaines telles que les cheveux mettant en oeuvre les compositions tinctoriales telles que définies précédemment.

Selon ce procédé, on applique sur les fibres au moins une composition tinctoriale (A) telle que définie ci-dessus avec laccase ou une composition tinctoriale prête à l'emploi telle que définie ci-avant pendant un temps suffisant pour développer la coloration désirée, après quoi on rince, on lave éventuellement au shampooing, on rince à nouveau et on sèche. Le temps nécessaire au développement de la coloration sur les fibres kératiniques est généralement compris entre 3 et 60 minutes et encore plus précisément 5 et 40 minutes.

L'application de la composition tinctoriale prête à l'emploi peut avoir lieu notamment à une température comprise entre la température ambiante (20° C) et 60° C, et préférentiellement entre 35 et 50 °C.

Selon une forme de réalisation particulière de l'invention, le procédé comporte une étape préliminaire consistant à stocker sous forme séparée, d'une part, une composition (A) telle que définie ci-dessus et d'autre part, une composition (B) définie ci-avant puis à procéder à leur mélange au moment de l'emploi avant d'appliquer ce mélange sur les fibres kératiniques.

L'invention a également pour objet des dispositifs de teinture à plusieurs compartiments, ou "kits" de teinture comportant au moins deux compartiments, dont l'un contient une composition (A) contenant au moins un précurseur de colorant d'oxydation et éventuellement un ou plusieurs coupleurs et, à titre d'agent réducteur, de la N-acétylcystéine, et un autre contient une composition oxydante (B) contenant au moins une laccase. Ces dispositifs peuvent être équipés d'un moyen permettant de délivrer sur les cheveux le mélange souhaité, tel que les dispositifs décrits dans le brevet FR-2 586 913.

Il est bien entendu que la description qui précède n'a été donnée qu'à titre purement illustratif et non limitatif et que des variantes ou des modifications peuvent y être apportées dans le cadre de la présente invention.

Des exemples concrets illustrant l'invention vont maintenant être donnés sans pour autant présenter un caractère limitatif.

## EXEMPLES COMPARATIFS

On prépare les compositions tinctoriales suivantes (teneurs en grammes):

| EXEMPLE | 1(*) | 2 (*) | 3 | 4(*) |
|---|---|---|---|---|
| Paraphénylènediamine ($10^{-3}$ mole) | 0,108 g | 0,108 g | 0,108 g | 0,108 g |
| 1-méthyl-2 hydroxy-4 amino benzène ($10^{-3}$ mole) | 0,123 g | 0,123 g | 0,123 g | 0,123 g |
| N-acétyl-L-cystéine | - | - | 0,1 g | - |
| Glucose | - | 5 g | - | - |
| Acide érythorbique | - | - | - | 0,3 g |
| Tampon phosphate commercialisé sous la dénomination Titrisol par la société Merck | pH 7 | pH 7 | pH 7 | pH 7 |
| Eau déminéralisée q.s.p. | (100-x) g | (100-x) g | (100-x) g | (100-x) g |

(*) Exemples ne faisant pas partie de l'invention

On ajoute au moment de l'emploi x g d'une solution de laccase pour obtenir une composition tinctoriale finale ayant une concentration de laccase égale à $10^7$ unités u.
Puis, chacune des compositions tinctoriales obtenues a été appliquée sur des mèches de cheveux gris naturels à 90% de blancs, à raison de 5 g de composition par g de cheveux, pendant 30 minutes à 40° C. Les cheveux ont ensuite été rincés, lavés au shampooing, rincés à nouveau puis séchés.
Les cheveux teints avec les compositions 1*, 2* et 3 présentaient la même nuance (pourpre rouge moyen).
Afin de déterminer de façon plus précise la montée de la coloration, la couleur des mèches a été évaluée avant et après la teinture dans le système MUNSELL, au moyen d'un colorimètre MINOLTA CM-2002®.
Selon la notation MUNSELL, une couleur est définie par l'expression H V/C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.
La différence entre la couleur de la mèche avant la teinture et la couleur de la mèche après la teinture exprime la puissance de la coloration et a été calculée en appliquant la formule de NICKERSON:

$$\Delta E = 0{,}4\ C0\Delta H + 6\Delta V + 3\Delta C$$

telle que décrite par exemple dans "Couleur, Industrie et Technique"; pages 14-17 ; vol. n° 5; 1978.
Dans cette formule, $\Delta E$ représente la différence de couleur entre deux mèches, $\Delta H$, $\Delta V$ et $\Delta C$ représentent la variation en valeur absolue des paramètres H, V et C et $C_0$ représente la pureté de la mèche par rapport à laquelle on désire évaluer la différence de couleur.
Plus la valeur de $\Delta E$ est élevée et plus la coloration est puissante.
Les résultats sont donnés dans le tableau ci-dessous:

| Composition | ΔE |
|---|---|
| 1 (*) | 30,94 |
| 2 (*) | 31,29 |
| 3 | 32,07 |
| 4 (*) | 5,75 |

Ces résultats montrent que la composition 2 ne faisant pas partie de l'invention et la composition 3 conforme à l'invention conduisent à une coloration aussi puissante que la composition 1 ne faisant pas partie de l'invention et qui ne contient pas d'agent réducteur. En revanche, la coloration obtenue avec la composition 4* utilisant l'acide érythorbique comme réducteur est faible. Ainsi, l'utilisation de N-acétylcystéine ne freine pas la montée de la coloration, et permet d'obtenir des colorations aussi puissantes que celles obtenues sans agent réducteur.
Les compositions tinctoriales 2* et 3 mentionnées ci-dessus ont également été conservées à une température ambiante de 22°C ± 2°C pendant 2 semaines.
Les mêmes colorations que celles précédemment décrites ont ensuite été réalisées.
Les résultats sont donnés dans le tableau suivant:

| Composition | ΔE |
|---|---|
| 2 (*) | 13,58 |
| 3 | 31,35 |

Ainsi, seule l'utilisation en tant que réducteur de la N-acétylcystéine permet de réduire l'oxydation des précurseurs de coloration tout en ne modifiant pas dans le temps la montée de la coloration sur les fibres.

## Revendications

1. Procédé de teinture des fibres kératiniques et en particulier des fibres kératiniques humaines telles que les cheveux, **caractérisé par le fait qu'**il consiste:

   - à appliquer sur les fibres une composition de teinture (A) contenant, dans un milieu approprié pour la teinture, au moins un précurseur de colorant d'oxydation et, éventuellement, un ou plusieurs coupleurs et, à titre d'agent réducteur, de 0,01 à 0,25% en poids, par rapport au poids total de la composition (A), de N-acétylcystéine, et
   - à révéler en présence d'air la couleur en milieu alcalin, neutre ou acide à l'aide d'au moins une laccase incorporée dans la composition (A) ou dans une composition (B),

   les compositions (A) et (B) étant mélangées immédiatement avant l'emploi ou appliquées l'une après l'autre sur les fibres kératiniques.

2. Procédé selon la revendication 1, dans lequel la laccase est choisie parmi les laccases d'origine végétale, d'origine animale, d'origine fongique, d'origine bactérienne, ou obtenues par biotechnologie.

3. Procédé selon l'une quelconque des revendications 1 et 2, dans lequel la laccase est choisie parmi celles produites par des végétaux effectuant la synthèse chlorophyllienne.

4. Procédé selon la revendication 3, dans lequel la laccase est choisie parmi celles extraites des Anacardiacées ou des Podocarpacées, des Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

5. Procédé selon la revendication 2, dans lequel la laccase est choisie parmi celles issues de Pyricularia orizae, de Polyporus versicolor, de Rhizoctonia praticola, de Rhus vemicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

6. Procédé selon l'une quelconque des revendications 1-5, dans lequel la laccase est présente dans des quantités allant de 0,5 à 3000 lacu, ou de 1000 à $6.10^7$ unités u; ou de 20 à $3.10^6$ unités ulac, pour 100g de composition prête à l'emploi.

7. Procédé selon l'une quelconque des revendications 1-6, dans lequel les précurseurs de colorants d'oxydation de la composition (A) sont choisis parmi les ortho- ou para-phénylènediamines, les bis-phénylalkylènediamines, les ortho- ou para-aminophénols, et les bases hétérocycliques ainsi que les sels d'addition d'un acide de ces composés.

8. Procédé selon la revendication 7, dans lequel les précurseurs de colorants d'oxydation sont présents à raison de 0,0005 à 12 % en poids par rapport au poids total de la composition (A).

**9.** Procédé selon l'une quelconque des revendications 1-8, dans lequel les coupleurs de la composition (A) sont choisis parmi les méta-phénylènediamines, les méta-aminophénols, les méta-diphénols, les coupleurs hétérocycliques et les sels d'addition d'un acide de ces composés.

**10.** Procédé selon la revendication 9, dans lequel les coupleurs sont présents à raison de 0,0001 à 10 % en poids par rapport au poids total de la composition (A).

**11.** Procédé selon les revendications 7 et 9, dans lequel les sels d'addition d'un acide des précurseurs de colorants d'oxydation et des coupleurs sont choisis parmi les chlorhydrates, les bromhydrates, les sulfates, les tartrates, les lactates et les acétates.

**12.** Procédé selon l'une quelconque des revendications 1-11, dans lequel la composition (A) contient en outre des colorants directs.

**13.** Procédé selon l'une quelconque des revendications 1-12, dans lequel la composition (A) et/ou (B) contient en outre au moins un polymère substantif cationique ou amphotère.

**14.** Procédé selon la revendication 13, dans lequel le polymère substantif est un polymère poly(ammonium quaternaire) constitué de motifs récurrents répondant à la formule (IV) suivante :

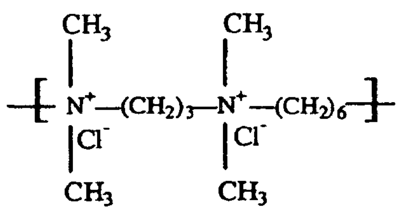

(IV)

ayant une masse molaire moyenne en poids, déterminée par chromatographie par perméation de gel, comprise entre 9500 et 9900.

**15.** Procédé selon la revendication 13, dans lequel le polymère substantif est un polymère poly(ammonium quaternaire) constitué de motifs récurrents répondant à la formule (V) suivante :

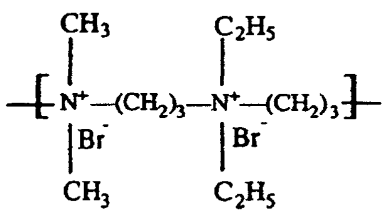

(V)

ayant une masse molaire moyenne en poids, déterminée par chromatographie par perméation de gel, d'environ 1200.

**16.** Procédé selon l'une quelconque des revendications 1-15, dans lequel la composition (A) contient, en outre, un ou plusieurs adjuvants choisis parmi les agents séquestrants, les agents de conditionnement du cheveu, notamment des silicones, les agents conservateurs, les agents opacifiants et les agents tensio-actifs anioniques, non-ioniques, amphotères ou leurs mélanges.

**17.** Procédé selon l'une quelconque des revendications 1-16, dans lequel la valeur du pH de la composition prête à l'emploi est comprise entre 3 et 11, de préférence entre 4 et 9, et encore plus préférentiellement entre 6 et 8.

**18.** Composition de teinture d'oxydation, stockée à l'abri de l'air, contenant, dans un milieu approprié pour la teinture,

- au moins un précurseur de colorant d'oxydation et, éventuellement, un ou plusieurs coupleurs,
- de 0,01 à 0,25 % en poids, par rapport au poids total de la composition, de N-acétylcystéine, à titre d'agent réducteur, et

- au moins une laccase.

**19.** Composition de teinture d'oxydation prête à l'emploi susceptible d'être obtenue par mélange

- d'une composition (A) contenant, dans un milieu approprié pour la teinture, au moins un précurseur de colorant d'oxydation et, éventuellement, un ou plusieurs coupleurs et, à titre d'agent réducteur, de 0,01 à 0,25 % en poids, par rapport au poids total de la composition (A), de N-acétylcystéine, et
- d'une composition (B) contenant au moins une laccase.

**20.** Procédé de teinture des fibres kératiniques et en particulier des fibres kératiniques humaines telles que les cheveux selon la revendication 1, **caracterisé par le fait que** l'on applique sur les fibres au moins une composition tinctoriale (A) avec laccase selon la revendication 18 ou une composition tinctoriale prête à l'emploi selon la revendication 19, pendant un temps suffisant pour développer la coloration désirée.

**21.** Procédé selon la revendication 20, dans lequel l'application de la composition tinctoriale prête à l'emploi est réalisée à une température comprise entre 20 et 60 °C et préférentiellement entre 35 et 50 °C.

**22.** Dispositif à plusieurs compartiments, ou "kit", pour la teinture des fibres kératiniques et en particulier des fibres kératiniques humaines telles que les cheveux, **caractérisé par le fait qu'**il comporte au moins deux compartiments dont l'un contient une composition (A) contenant au moins un précurseur de colorant d'oxydation et éventuellement un ou plusieurs coupleurs et, à titre d'agent réducteur, de la N-acétylcystéine, et un autre compartiment contient une composition oxydante (B) contenant au moins une laccase.

## Patentansprüche

**1.** Verfahren zum Färben von Keratinfasern und insbesondere menschlichen Keratinfasern, wie dem Haar, **dadurch gekennzeichnet, dass** es darin besteht:

- auf die Fasern eine Farbmittelzusammensetzung (A) aufzutragen, die in einem zum Färben geeigneten Medium mindestens ein Farbstoffvorprodukt eines Oxidationsfarbstoffes, gegebenenfalls einen oder mehrere Kuppler und als Reduktionsmittel 0,01 bis 0,25 Gew.-% N-Acetylcystein, bezogen auf das Gesamtgewicht der Zusammensetzung (A), enthält, und
- die Farbe in Gegenwart von Luft in einem alkalischen, neutralen oder sauren Medium mit mindestens einer Laccase zu entwickeln, die in der Zusammensetzung (A) oder in einer Zusammensetzung (B) vorliegt,

wobei die Zusammensetzungen (A) und (B) unmittelbar vor der Anwendung vermischt oder nacheinander auf die Keratinfasern aufgetragen werden.

**2.** Verfahren nach Anspruch 1, wobei die Laccase unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt ist.

**3.** Verfahren nach einem der Ansprüche 1 und 2, wobei die Laccase unter den Laccasen ausgewählt ist, die von Pflanzen gebildet werden, die Chlorophyll synthetisieren.

**4.** Verfahren nach Anspruch 3, wobei die Laccase unter den Laccasen ausgewählt ist, die aus Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica und Pistacia palaestina extrahiert wurden.

**5.** Verfahren nach Anspruch 2, wobei die Laccase unter den Laccasen ausgewählt ist, die von Pyricularia orizae, Polyporus versicolor, Rhizoctonia praticola, Rhus vernicifera, Scytalidium, Polyporus pinsitus, Myceliophtora thermophila, Rhizoctonia solani, Trametes versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus

sphinctrinus, Schizophyllum commune, Dichomitus squalens und deren Varianten stammen.

**6.** Verfahren nach einem der Ansprüche 1 bis 5, wobei die Laccase in einem Mengenanteil von 0,5 bis 3000 lacu, 1000 bis $6 \cdot 10^7$ Einheiten u oder 20 bis $3 \cdot 10^6$ Einheiten ulac auf 100 g der gebrauchsfertigen Zusammensetzung enthalten ist.

**7.** Verfahren nach einem der Ansprüche 1 bis 6, wobei die Farbstoffvorprodukte von Oxidationsfarbstoffen der Zusammensetzung (A) unter den o- oder p-Phenylendiaminen, Bisphenylalkylendiaminen, o- oder p-Aminophenolen und den heterocyclischen Basen und den Additionssalzen dieser Verbindungen mit einer Säure ausgewählt sind.

**8.** Verfahren nach Anspruch 7, wobei die Farbstoffvorprodukte von Oxidationsfarbstoffen in einer Menge von 0,0005 bis 12 Gew.-%, bezogen auf das Gesamtgewicht der Zusammensetzung (A), enthalten sind.

**9.** Verfahren nach einem der Ansprüche 1 bis 8, wobei die Kuppler der Zusammensetzung (A) unter den m-Phenylendiaminen, m-Aminophenolen, m-Dihydroxybenzolen und den heterocyclischen Kupplern und den Additionssalzen dieser Verbindungen mit einer Säure ausgewählt sind.

**10.** Verfahren nach Anspruch 9, wobei die Kuppler in einer Menge von 0,0001 bis 10 Gew.-%, bezogen auf das Gesamtgewicht der Zusammensetzung (A), enthalten sind.

**11.** Verfahren nach den Ansprüchen 7 und 9, wobei die Additionssalze der Farbstoffvorprodukte von Oxidationsfarbstoffen und Kuppler mit einer Säure unter den Hydrochloriden, Hydrobromiden, Sulfaten, Tartraten, Lactaten und Acetaten ausgewählt sind.

**12.** Verfahren nach einem der Ansprüche 1 bis 11, wobei die Zusammensetzung (A) ferner Direktfarbstoffe enthält.

**13.** Verfahren nach einem der Ansprüche 1 bis 12, wobei die Zusammensetzung (A) und/oder (B) außerdem mindestens ein kationisches oder amphoteres substantives Polymer enthält.

**14.** Verfahren nach Anspruch 13, wobei das substantive Polymer ein Poly(quartäres Ammonium)-Polymer ist, das aus wiederkehrenden Einheiten der folgenden Formel (IV) besteht:

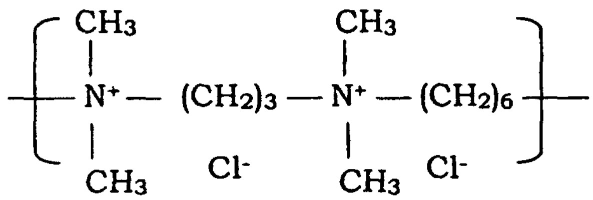

(IV),

das eine mit Gelpermeations-Chromatographie ermittelte, gewichtsmittlere Molmasse von 9500 bis 9900 besitzt.

**15.** Verfahren nach Anspruch 13, wobei das substantive Polymer ein Poly(quartäres Ammonium)-Polymer ist, das aus wiederkehrenden Einheiten der folgenden Formel (V) besteht:

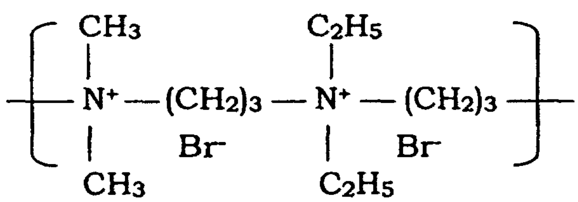

(V),

das eine mit Gelpermeations-Chromatographie ermittelte, gewichtsmittlere Molmasse von etwa 1200 besitzt.

**16.** Verfahren nach einem der Ansprüche 1 bis 15, wobei die Zusammensetzung (A) ferner einen oder mehrere Zusatzstoffe enthält, die unter den Maskierungsmitteln, Konditioniermitteln für die Haare, insbesondere Siliconen, Konservierungsmitteln, Trübungsmitteln und anionischen, nichtionischen oder amphoteren grenzflächenaktiven Stoffen oder deren Gemischen ausgewählt sind.

**17.** Verfahren nach einem der Ansprüche 1 bis 16, wobei der pH-Wert der gebrauchsfertigen Zusammensetzung im Bereich von 3 bis 11, vorzugsweise 4 bis 9 und noch bevorzugter 6 bis 8 liegt.

**18.** Zusammensetzung zum oxidativen Färben, die unter Luftabschluss aufbewahrt wird und die in einem zum Färben geeigneten Medium

- mindestens ein Farbstoffvorprodukt eines Oxidationsfarbstoffes und gegebenenfalls einen oder mehrere Kuppler,
- als Reduktionsmittel 0,01 bis 0,25 Gew.-% N-Acetylcystein, bezogen auf das Gesamtgewicht der Zusammensetzung, und
- mindestens eine Laccase enthält.

**19.** Gebrauchsfertige Zusammensetzung zum oxidativen Färben, erhältlich durch Mischen

- einer Zusammensetzung (A), die in einem zum Färben geeigneten Medium mindestens ein Farbstoffvorprodukt eines Oxidationsfarbstoffes, gegebenenfalls einen oder mehrere Kuppler und als Reduktionsmittel 0,01 bis 0,25 Gew.-% N-Acetylcystein, bezogen auf das Gesamtgewicht der Zusammensetzung (A), enthält, und
- einer Zusammensetzung (B), die mindestens eine Laccase enthält.

**20.** Verfahren zum Färben von Keratinfasern und insbesondere menschlichen Keratinfasern, wie dem Haar, nach Anspruch 1, **dadurch gekennzeichnet, dass** auf die Fasern mindestens eine Farbmittelzusammensetzung (A) mit Laccase nach Anspruch 18 oder eine gebrauchsfertige Zusammensetzung nach Anspruch 19 während einer Zeitspanne, die zur Entwicklung der gewünschten Färbung ausreichend ist, aufgebracht wird.

**21.** Verfahren nach Anspruch 20, wobei die gebrauchsfertige Farbmittelzusammensetzung bei einer Temperatur von 20 bis 60 °C und vorzugsweise 35 bis 50 °C hergestellt wird.

**22.** Vorrichtung mit mehreren Abteilungen oder "Kit" zum Färben von Keratinfasern und insbesondere menschlichen Keratinfasern, wie dem Haar, **dadurch gekennzeichnet, dass** sie mindestens zwei Abteilungen enthält, wobei eine Abteilung eine Zusammensetzung (A), die mindestens ein Farbstoffvorprodukt eines Oxidationsfarbstoffes, gegebenenfalls einen oder mehrere Kuppler und als Reduktionsmittel das N-Acetylcystein enthält, und eine zweite Abteilung eine oxidierende Zusammensetzung (B) enthält, in der mindestens eine Laccase enthalten ist.

## Claims

**1.** Process for dyeing keratin fibres, and in particular human keratin fibres such as the hair, **characterized in that** it consists:

- in applying to the fibres a dye composition (A) containing, in a medium which is suitable for dyeing, at least one oxidation dye precursor and, optionally, one or more couplers and, as reducing agent, from 0.01% to 0.25% by weight of N-acetylcysteine relative to the total weight of the composition (A), and

- in developing the colour in the presence of air in alkaline, neutral or acidic medium using at least one laccase incorporated into the composition (A) or into a composition (B),

the compositions (A) and (B) being mixed together immediately before use or applied one after the other to the keratin fibres.

**2.** Process according to Claim 1, in which the laccase is chosen from laccases of plant origin, of animal origin, of fungal origin and of bacterial origin, or obtained by biotechnology.

**3.** Process according to either of Claims 1 and 2, in which the laccase is chosen from those produced by plants which

carry out chlorophyll synthesis.

4. Process according to Claim 3, in which the laccase is chosen from those extracted from Anacardiacea plants or Podocarpacea plants, from Rosmarinus off.; from Solanum tuberosum; from Iris sp.; from Coffea sp.; from Daucus carrota; from Vinca minor; from Persea americana; from Catharenthus roseus; from Musa sp.; from Malus pumila; from Gingko biloba; from Monotropa hypopithys (Indian pipe), from Aesculus sp.; from Acer pseudoplatanus; from Prunus persica and from Pistacia palaestina.

5. Process according to Claim 2, in which the laccase is chosen from those obtained from Pyricularia orizae, from Polyporus versicolor, from Rhizoctonia praticola, from Rhus vernicifera, from Scytalidium, from Polyporus pinsitus, from Myceliophthora thermophila, from Rhizoctonia solani, from Trametes versicolor, from Fomes fomentarius, from Chaetomium thermophile, from Neurospora crassa, from Coriolus versicol, from Botrytis cinerea, from Rigidoporus lignosus, from Phellinus noxius, from Pleurotus ostreatus, from Aspergillus nidulans, from Podospora anserina, from Agaricus bisporus, from Ganoderma lucidum, from Glomerella cingulata, from Lactarius piperatus, from Russula delica, from Heterobasidion annosum, from Thelephora terrestris, from Cladosporium cladosporioides, from Cerrena unicolor, from Coriolus hirsutus, from Ceriporiopsis subvermispora, from Coprinus cinereus, from Panaeolus papilionaceus, from Panaeolus sphinctrinus, from Schizophyllum commune, from Dichomitius squalens, and from variants thereof.

6. Process according to any one of Claims 1 to 5, in which the laccase is present in amounts ranging from 0.5 to 3 000 lacu, or from 1 000 to 6 $10^7$ u units; or from 20 to 3 $10^6$ ulac units, per 100 g of ready-to-use composition.

7. Process according to any one of Claims 1 to 6, in which the oxidation dye precursors of the composition (A) are chosen from ortho- and para-phenylenediamines, bis(phenyl)alkylenediamines, ortho- and para-aminophenols, and heterocyclic bases, and also addition salts of these compounds with an acid.

8. Process according to Claim 7, in which the oxidation dye precursors are present in a proportion of from 0.0005% to 12% by weight relative to the total weight of the composition (A).

9. Process according to any one of Claims 1 to 8, in which the couplers of the composition (A) are chosen from meta-phenylenediamines, meta-aminophenols, meta-diphenols and heterocyclic couplers, and the addition salts of these compounds with an acid.

10. Process according to Claim 9, in which the couplers are present in a proportion of from 0.0001% to 10% by weight relative to the total weight of the composition (A).

11. Process according to Claims 7 and 9, in which the addition salts of the oxidation dye precursors and of the couplers with an acid are chosen from the hydrochlorides, hydrobromides, sulphates, tartrates, lactates and acetates.

12. Process according to any one of Claims 1 to 11, in which the composition (A) also contains direct dyes.

13. Process according to any one of Claims 1 to 12, in which the composition (A) and/or (B) also contains at least one cationic or amphoteric substantive polymer.

14. Process according to Claim 13, in which the substantive polymer is a poly(quaternary ammonium) polymer consisting of repeating units corresponding to formula (IV) below:

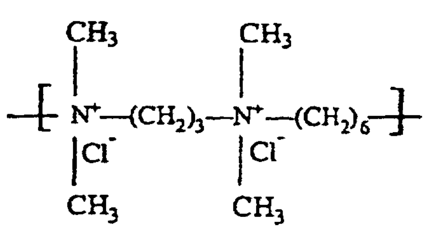

(IV)

having a weight-average molar mass as determined by gel permeation chromatography of between 9500 and 9900.

**15.** Process according to Claim 13, in which the substantive polymer is a poly(quaternay ammonium) polymer consisting of repeating units corresponding to formula (V) below:

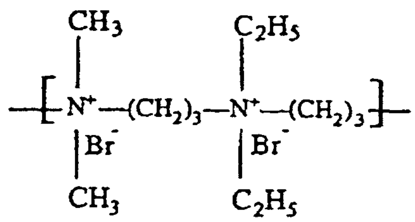

(V)

having a weight-average molar mass as determined by gel permeation chromatography of approximately 1200.

**16.** Process according to any one of Claims 1 to 15, in which the composition (A) also contains one or more adjuvants chosen from sequestering agents, hair conditioners, in particular silicones, preserving agents, opacifiers and anionic, nonionic or amphoteric surfactants, or mixtures thereof.

**17.** Process according to any one of Claims 1 to 16, in which the pH value of the ready-to-use composition is between 3 and 11, preferably between 4 and 9 and even more preferably between 6 and 8.

**18.** Oxidation dyeing composition stored sheltered from the air and comprising, in a medium which is suitable for dyeing,

- at least one oxidation dye precursor and, optionally, one or more couplers,
- from 0.01% to 0.25% by weight of N-acetylcysteine relative to the total weight of the composition, as reducing agent, and
- at least one laccase.

**19.** Ready-to-use oxidation dyeing composition obtainable by mixing

- a composition (A) containing, in a medium which is suitable for dyeing, at least one oxidation dye precursor and, optionally, one or more couplers and, as reducing agent, from 0.01% to 0.25% by weight of N-acetylcysteine relative to the total weight of the composition (A), and
- a composition (B) containing at least one laccase.

**20.** Process for dyeing keratin fibres, and in particular human keratin fibres such as the hair according to Claim 1 **characterized in that** at least one dye composition (A) with laccase according to Claim 18 or a ready-to-use dye composition according to Claim 19 is applied to the fibres for a period which is sufficient to develop the desired coloration.

**21.** Process according to Claim 20, in which the application of the ready-to-use dye composition is carried out at a temperature of between 20°C and 60°C and preferably between 35°C and 50°C.

**22.** Multi-compartment device, or Akit@, for dyeing keratin fibres, and in particular human keratin fibres such as the hair, **characterized in that** it comprises at least two compartments, one of which contains a composition (A) containing at least one oxidation dye precursor and optionally one or more couplers and, as reducing agent, N-acetylcysteine, and another compartment contains an oxidizing composition (B) containing at least one laccase.